# EUROPEAN PATENT APPLICATION

(11) **EP 4 567 708 A1**
(43) Date of publication of application: **11.06.2025**
(21) Application number: 23849696.2
(22) Date of filing: 04.04.2023
(51) Int. Cl.: G06Q 30/0601, G06Q 50/12

(54) **INFORMATION PROVISION SYSTEM FOR FOOD-SERVING STORES**

(30) Priority: 05.08.2022 JP 2022125521
(71) Applicant: Hanabeni Inc., Tokyo 140-0001 (JP)
(72) Inventor: TAKARA Haruki, Tokyo 140-0001 (JP)
(74) Representative: Danubia Patent & Law Office LLC
(86) International application number: PCT/JP2023/014007
(87) International publication number: WO 2024/029133

(57) **Abstract**

This is an information provision system for food service establishments designed to support maintaining a balanced diet while promoting a diverse dietary lifestyle. The system includes a dish information storage unit that stores information about dishes offered by food service establishments, linked to their nutritional information. It also has a meal information storage unit that records user meal information, including nutritional details of dishes the user has consumed in the past or plans to consume in the future, associated with the date or period of consumption.

Using the user meal information and the nutritional information of the dishes, a nutritional balance assessment unit identifies recommended dishes stored in the dish information storage unit that are nutritionally appropriate for the user. The system further includes a communication control unit that transmits information about these recommended dishes, along with details of the food service establishments offering them, to the user's information terminal. This system helps food service establishments provide recommendations tailored to the nutritional needs of individual users.

## Description

### Technical Field

The present invention relates to an information provision system for food service establishments.

### Background Art

With the widespread use of information devices such as smartphones and car navigation systems, services that display information about restaurants and cafes operating in a predetermined area are being provided. However, except for frequently used chain stores or regular favorite places, users often do not obtain sufficient information about the contents of food and beverages offered by restaurants or cafes displayed on these devices.

On the other hand, in recent years, with the rise of health consciousness, an increasing number of people are concerned about the calories, salt content, carbohydrate content, and other nutritional aspects of dishes even when eating out. As a system for providing information about the nutritional aspects of dishes served at restaurants when eating out, for example, it has been proposed to indicate whether ingredients that users should restrict are included in the menu items of target restaurants (see Patent Document 1, etc.).

However, since meals are one of the important pleasures in life, if users judge whether or not to consume a dish in a uniform manner based solely on its nutritional information, as in the conventional approach, it excessively limits the user's enjoyment of daily life.

### Prior Art Documents

### Patent Documents

**[Patent Document 1]** Japanese Patent Laid-Open Publication No. 2011-248706

### Summary of the Invention

### Problems to be Solved by the Invention

The present invention relates to an information provision system for food service establishments that can support maintaining a nutritional balance while engaging in a diverse diet.

### Means for Solving the Problems

The information provision system for food service establishments according to the present invention comprises:
A dish information storage unit that stores dishes provided by food service establishments in association with dish nutritional information, which is information about the nutrition of the dishes;
A meal information storage unit that stores user meal information, which includes information about the nutrition of dishes that a predetermined user has consumed in the past or plans to consume in the future, associated with the date and time or period of consumption;
A nutritional balance determination unit that, using the user meal information and the dish nutritional information, extracts recommended dishes suitable for the user from the dishes stored in the dish information storage unit from a nutritional standpoint;
A communication control unit that transmits information about the recommended dishes and the recommended food service establishments providing these dishes to the user's information terminal.

The information provision system for food service establishments according to the present invention not only stores dish nutritional information but also stores information about dishes that users have consumed in the past or plan to consume in the future, and by using these, can provide users with information about diverse recommended dishes that are not uniform, while taking into consideration the users' health. Moreover, by providing information about food service establishments and dishes that users have not visited or tried, the system can support users' diverse dietary lifestyles.

Furthermore, for example, the information provision system for food service establishments according to the present invention may include a utilization information acquisition unit that obtains utilization information indicating that the user has consumed or plans to consume the dishes stored in the dish information storage unit, and a meal information updating unit that updates the user meal information stored in the meal information storage unit based on the utilization information and the dish nutritional information stored in the dish information storage unit.

According to such an information provision system for food service establishments, since the user meal information is automatically updated based on partnered restaurant usage information, users can receive information about recommended food service establishments and recommended dishes from the system without having to input information about the dishes themselves.

For example, the information provision system for food service establishments according to the present invention may include a regional information storage unit that stores geographical information, including the addresses of the food service establishments and information about their surrounding areas, and a location information acquisition unit that obtains the location information of the user's information terminal.
The communication control unit may, when at least one of the food service establishments within a predetermined range of the information terminal is included recommended food service establishments, transmit the geographical information of the recommended food service establishments within the predetermined range of the information terminal to the user's information terminal, distinguishing it from the geographical information of other food service establishments within the predetermined range that are not recommended food service establishments.

By transmitting information about food service establishments using the user's location information, users can obtain information about food service establishments that are geographically convenient to use. Additionally, by distinguishing recommended food service establishments from non-recommended ones when providing information about food service establishments, the system can increase users' choices and facilitate their decision-making.

Additionally, for example, the communication control unit may receive order information indicating the user's intention to order the recommended dishes from the user's information terminal and transmit the order information to the recommended food service establishments providing the recommended dishes.

According to such an information provision system for food service establishments, users can manage everything from searching for dishes to placing orders in a centralized manner.

Furthermore, for example, the communication control unit may receive payment information indicating the user's intention to settle the order along with the order information and transmit the payment information to a predetermined payment institution.

According to such an information provision system for food service establishments, users can manage everything from searching for dishes to placing orders and settling payments in a centralized manner.

Additionally, for example, the communication control unit may transmit information about the recommended dishes and the recommended food service establishments, along with information about the reasons for recommending the recommended dishes from a nutritional standpoint, to the user's information terminal.

According to such an information provision system for food service establishments, users can know the reasons for recommending the recommended dishes and learn about what to pay attention to nutritionally when having meals.

Furthermore, for example, the communication control unit may transmit information about the recommended dishes and the recommended food service establishments, along with information about an index indicating the degree to which the recommended dishes are suitable for the user from a nutritional standpoint, to the user's information terminal.

According to such an information provision system for food service establishments, users can obtain more useful information for deciding whether to select the dishes by viewing the index of the recommended dishes.

Also, for example, the information provision system for food service establishments according to the present invention may include a regional information storage unit that stores geographical information, such as the addresses of the food service establishments and information about their surrounding areas, and a location information acquisition unit that obtains the location information of the user's information terminal. The nutritional balance determination unit may extract multiple recommended dishes and corresponding recommended food service establishments.
The communication control unit, when multiple recommended food service establishments within a predetermined range of the information terminal are extracted, may transmit the geographical information of the recommended food service establishments with the highest index among the multiple recommended food service establishments to the user's information terminal, distinguishing it from the geographical information of the other recommended food service establishments.

According to such an information provision system for food service establishments, if there are multiple recommended food service establishments that are easy to use, the user can easily recognize the information of the food service establishments with the highest index from the map, thereby obtaining more useful information to decide whether to select the dish.

Moreover, for example, the user meal information may include information about alcohol that the user has consumed in the past or plans to consume in the future.

By including information about alcohol consumption in the user meal information, the information provision system for food service establishments can, for example, propose dishes that consider the physical burden associated with alcohol consumption and provide more useful information for maintaining the user's health.

Additionally, for example, the user meal information may include information about the calories of the dishes that the user has consumed in the past or plans to consume in the future.

By including information about calorie intake in the user meal information, the information provision system for food service establishments can, for example, propose dishes that maintain an appropriate average daily calorie intake, thereby providing more useful information for the user's health maintenance.

### Brief Description of the Drawings

Figure 1 is a conceptual diagram is a conceptual diagram illustrating a server device and its peripheral devices that constitute the information provision system for food service establishments according to one embodiment of the present invention.
Figure 2 is a conceptual diagram showing the functions of the server device shown in Figure 1.
Figure 3 is a conceptual diagram showing the details of the user information management unit shown in Figure 2.
Figure 4 is a conceptual diagram showing the details of the meal information storage unit shown in Figure 3.
Figure 5 is a conceptual diagram showing the details of the store information management unit shown in Figure 2.
Figure 6 is a conceptual diagram showing the details of the dish information storage unit shown in Figure 5.
Figure 7 is a conceptual diagram showing the details of the nutritional balance determination unit shown in Figure 2.
Figure 8 is a flowchart showing an example of processing performed using the information system for food service establishments shown in Figure 1.
Figure 9 is a conceptual diagram showing an example of information displayed on the user information terminal shown in Figure 1.
Figure 10 is a conceptual diagram showing another example of user meal information stored in the meal information storage unit shown in Figure 3.

### Modes for Carrying Out the Invention

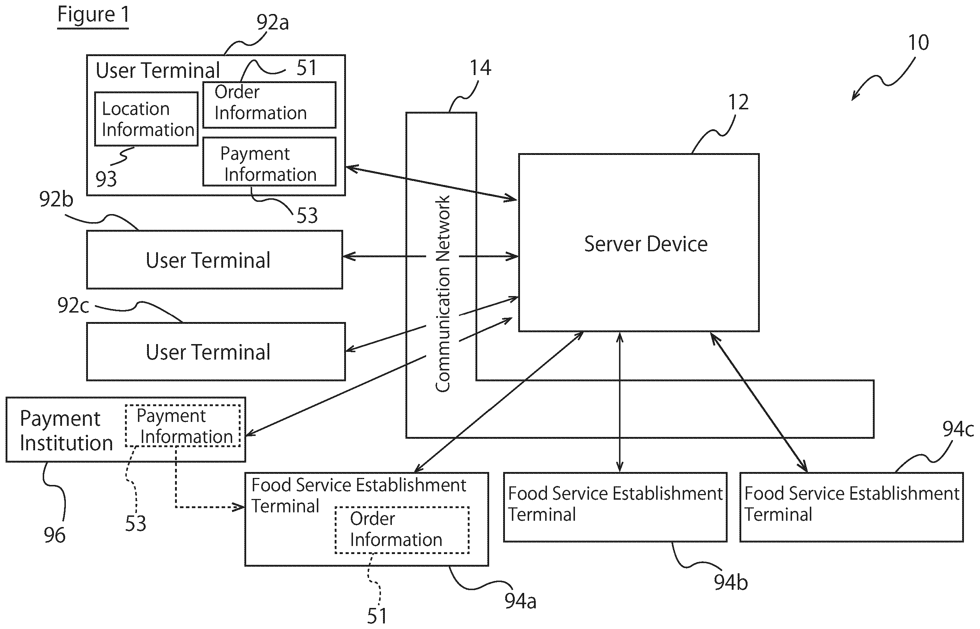

Figure 1 is a conceptual diagram showing a server device 12 and its peripheral devices that constitute the information provision system 10 for food service establishments according to an embodiment of the present invention. The server device 12 is connected in a communicable manner via a network communication network 14 such as the Internet to multiple user terminals 92a, 92b, 92c.

Additionally, the server device 12 is communicably connected via the network communication network 14 to food service establishment terminals 94a, 94b, 94c managed by food service establishments that provide dishes to customers, including the server device 12, and to a payment institution 96 that performs electronic settlement services that mediate the payment of fees by users to food service establishments.

As described later, in the information provision system 10 for food service establishments shown in Figure 1, the server device 12 provides information about recommended dishes that are suitable for users from a nutritional standpoint based on the dishes provided by food service establishments to the user terminals 92a, 92b, 92c, which are communication terminals used by users who are users of the information provision system 10. Also, users can order dishes provided by food service establishments, including recommended dishes, from the user terminals 92a, 92b, 92c or perform settlement of the orders.

Note that in the information provision system 10 shown in Figure 1, the server device 12 can communicate not only with user terminals 92a, 92b, 92c but also with food service establishment terminals 94a, 94b, 94c and payment institutions 96, but the server device 12 is not limited to such a form. For example, if the server device 12 does not have functions to order dishes or perform settlements, it suffices for the server device 12 to be able to communicate with user terminals 92a, 92b, 92c.

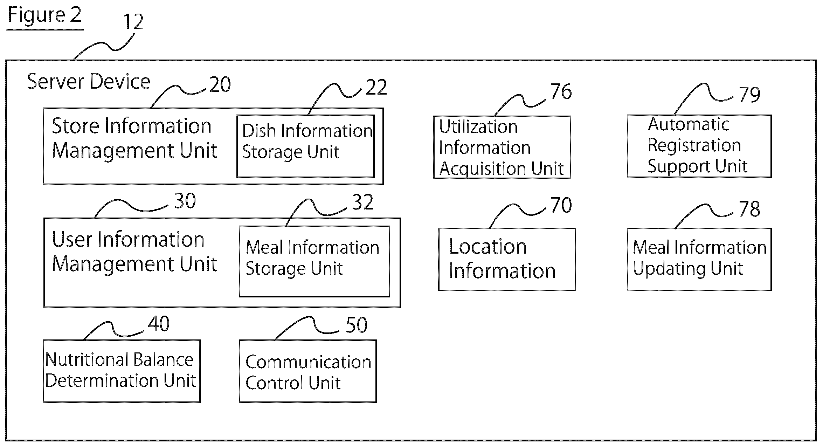

As shown in Figure 2, the server device 12 has a communication control unit 50, storage units (dish information storage unit 22, meal information storage unit 32), and an arithmetic unit, among others, and functions as a general computer with these units. In the description of the server device 12, commonalities with a general computer are omitted. Although the server device 12 shown in Figure 1 is cloud-based, the server device 12 is not limited to this; the server device 12 may also be on-premises.

The user terminals 92a, 92b, 92c shown in Figure 1 are information terminals used by users to receive information about food service establishments and dishes provided by these establishments from the information provision system 10. The user terminals 92a, 92b, 92c may include portable (mobile) information terminals such as mobile phones, smartphones, tablets, car navigation systems, etc., but are not limited to these and may include other communication-capable information terminals such as personal computers.

In the description of the information provision system 10, mainly the user terminal 92a is used as an example, but the user terminals 92b, 92c perform the same communications as the user terminal 92a. Also, the number of user terminals 92a, 92b, 92c accessing the server device 12 in the information provision system 10 is not particularly limited.

The food service establishment terminals 94a, 94b, 94c shown in Figure 1 are information terminals used by food service establishments that provide dishes to users, receiving information from the information provision system 10 that users receive on their user terminals 92a, 92b, 92c. The food service establishment terminals 94a, 94b, 94c may include computers, tablets, smartphones, etc., but are not limited to these. Here, food service establishments include restaurants and cafes that provide dishes to customers in-house, as well as *bento* shops, delivery restaurants, etc., that provide dishes to customers in take-out or delivery formats. Also, unless particularly limited, "dishes" include not only solid foods but also beverages.

Furthermore, in the description of the food service establishment terminals 94a, 94b, 94c, the food service establishment terminal 94a is mainly used as an example, but food service establishment terminals 94b, 94c also perform the same communications as the user terminal 94a. Also, the number of food service establishment terminals 94a, 94b, 94c accessing the server device 12 in the information provision system 10 is not particularly limited.

The payment institution 96 shown in Figure 1 is an information terminal of an institution that performs electronic payment services, such as credit card, debit card, electronic money, etc., mediating the payment of fees by users to food service establishments. These are not limited, and the payment institution 96 in the information provision system 10 is not limited to a single institution; it may include multiple payment institutions, and users may choose among them to perform payments.

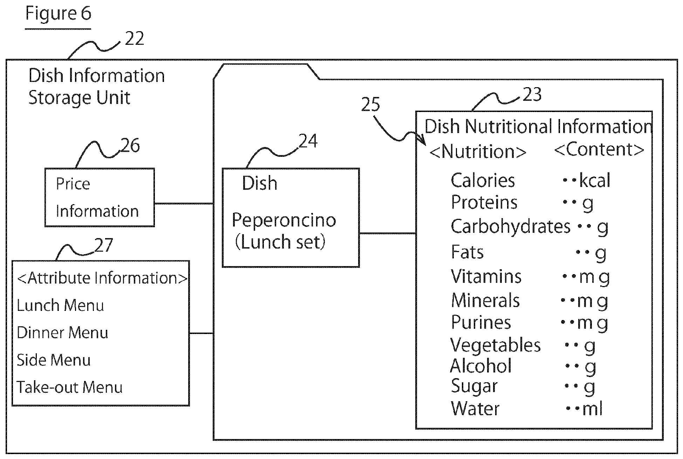

Figure 2 is a functional block diagram of the server device 12 shown in Figure 1. As shown in Figure 2, the server device 12 includes a store information management unit 20 that has the dish information storage unit 22, a user information management unit 30 that has the meal information storage unit 32, a nutritional balance determination unit 40, a communication control unit 50, and also a utilization information acquisition unit 76 that obtains information related to the user's use of food service establishments, a location information acquisition unit 70 that acquires location information 93 of the user's information terminal, a meal information updating unit 78 that updates the user meal information 33 stored in the meal information storage unit 32, and an automatic registration support unit 79 that supports the input of dish nutritional information 23 (see figure 6) to the dish information storage unit 22.

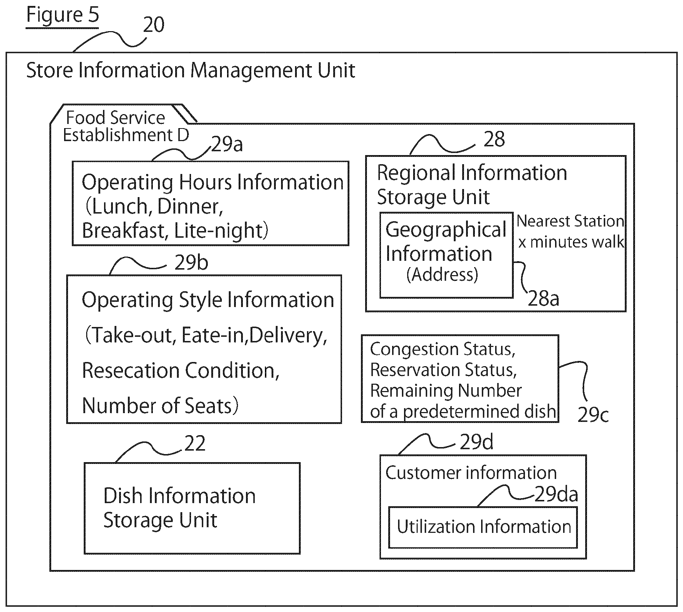

The store information management unit 20 shown in Figure 2 stores information about food service establishments and updates the information as needed. Figure 5 is a conceptual diagram showing the details of the store information management unit 20 shown in Figure 2. The store information management unit 20 stores information about food service establishments and updates it as needed. The store information management unit 20 stores information such as operating hours information 29a, operating style information 29b, real-time information 29c, and customer information 29d for each food service establishment. It also has the dish information storage unit 22 and the regional information storage unit 28.

The store information management unit 20 shown in Figure 5 stores various types of information related to food service establishments, distinguished by each establishment. The operating hours information 29a includes, for example, information about the operating hours of a given food service establishment, such as lunch hours, dinner hours, breakfast hours, late-night hours, business days, and regular holidays. The operating style information includes, for instance, details about the operational style of a given food service establishment, such as whether it offers takeout, dine-in, delivery, or accepts reservations.

Additionally, as shown in Figure 5, real-time information 29c includes information about the store's congestion status, reservation status, and remaining number of a predetermined dish (how many dishes can be provided on the day). Real-time information 29c is updated based on information obtained via the communication control unit 50 shown in Figure 2, such as order information 51 from users. Customer information 29d includes information about the ID of users who have used a predetermined food service establishment, the date and time of using the food service establishment, and the dishes ordered, etc. For example, customer information 29d includes information indicating whether the user has consumed a dish stored in the dish information storage unit 22 in the past or plans to consume it in the future, which is utilization information 29da. Utilization information 29da is obtained by the utilization information acquisition unit 76 shown in Figure 2. Note that information indicating whether the user has consumed a dish in the past or plans to consume it in the future includes, for example, order information or reservation information from users.

The regional information storage unit 28 shown in Figure 5 stores geographical information 28a, which is information about the address and surrounding geography of food service establishments. Geographical information 28a includes, in addition to addresses, information such as the nearest station and the required time from the nearest station. As described later, the server device 12 shown in Figure 2 can use the geographical information 28a of food service establishments to limit the search for recommended dishes by the nutritional balance determination unit 40 to dishes provided by establishments in a specific area that the user wishes to know about. It can also restrict the information provided to the user to food service establishments in a specific area.

As shown in Figure 5, the store information management unit 20 has the dish information storage unit 22. The dish information storage unit 22 stores dishes 24 provided by food service establishments in association with dish nutritional information 23, which includes information related to the nutrition of the dishes 25. Figure 6 is a conceptual diagram showing an example of the information stored by the dish information storage unit 22 shown in Figure 5. As shown in Figure 6, the dish information storage unit 22 stores dishes 24 (e.g., "Peperoncino (lunch set)" in the example shown in Figure 6) provided by food service establishments in association with dish nutritional information 23 (e.g., calories, proteins, carbohydrates, etc.) related to the nutrition of the dishes.

The dish information storage unit 22 preferably has dish nutritional information 23 for all dishes 24 that a predetermined food service establishment can provide as recommended dishes to users. Dish nutritional information 23 includes types and amounts of calories, proteins, carbohydrates, fats, vitamins, minerals, purines, vegetables, alcohol, sugar, salt, water, etc., contained in the dishes 24, but are not limited to these and may include other values recognized as meaningful for managing intake from a nutritional standpoint. Also, the dish nutritional information 23 related to the dish 24 can be one value or index (e.g., only calories) or multiple values or indices as shown in Figure 6. The types of nutritional information stored per dish 24 are not particularly limited.

As shown in Figure 6, in addition to dish nutritional information 23, the dish information storage unit 22 can store other information related to the dish 24, such as price information 26 regarding the price of the dish 24, and attribute information 27 indicating whether the dish 24 is a lunch menu, dinner menu, side menu, take-out menu, etc. The server device 12 shown in Figure 2 can use the price information 26 and attribute information 27 of the dishes 24 to limit the recommended dishes 42 explored by the nutritional balance determination unit 40 to dishes 24 that can be provided at the required time or format requested by users, or to limit information provision to dishes 24 within a predetermined price range for users.

The dish nutritional information 23 stored in the dish information storage unit 22 can be entered by inputting numerical data into the dish information storage unit 22 from the food service establishment terminal 94a shown in Figure 1 or by uploading correspondence data between dishes 24 and dish nutritional information 23 to the dish information storage unit 22. Additionally, the dish nutritional information 23 stored in the dish information storage unit 22 may also be automatically calculated and entered based on image data of dishes 24 by the automatic registration support unit 79 shown in Figure 2. The automatic registration support unit 79 can, for example, automatically recognize the dish nutritional information 23 and dish name of a dish 24 from image data uploaded to the server device 12 from a food service establishment terminal 51 (or user terminal 92a). It can then store the automatically recognized dish and its associated dish nutritional information 23 in the dish information storage unit 22.

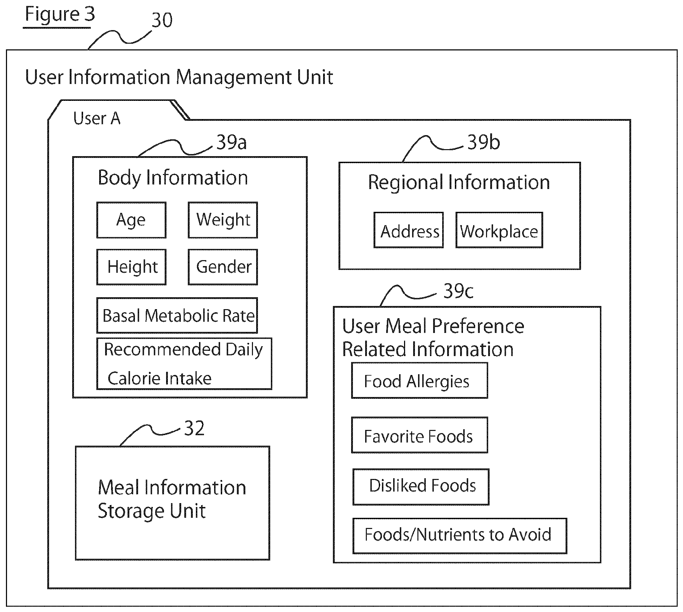

The user information management unit 30 shown in Figure 2 manages information about users who communicate with the server device 12 using user terminals 92a, 92b, and 92c. Figure 3 is a conceptual diagram showing the details of the user information management unit 30 depicted in Figure 2. In addition to the user meal information 33 described later, the user information management unit 30 manages individual user-specific information such as physical information 39a, regional information 39b, and meal preference-related information 39c, assigning identifiers such as user IDs for management.

As shown in Figure 3, the user information management unit 30 can store, for example, user body information 39a such as age, weight, height, gender, basal metabolic rate, and recommended daily calorie intake; user regional information 39b such as user address and workplace; and user meal preference related information 39c such as food allergies, favorite foods, disliked foods, foods/nutrients to avoid, etc.

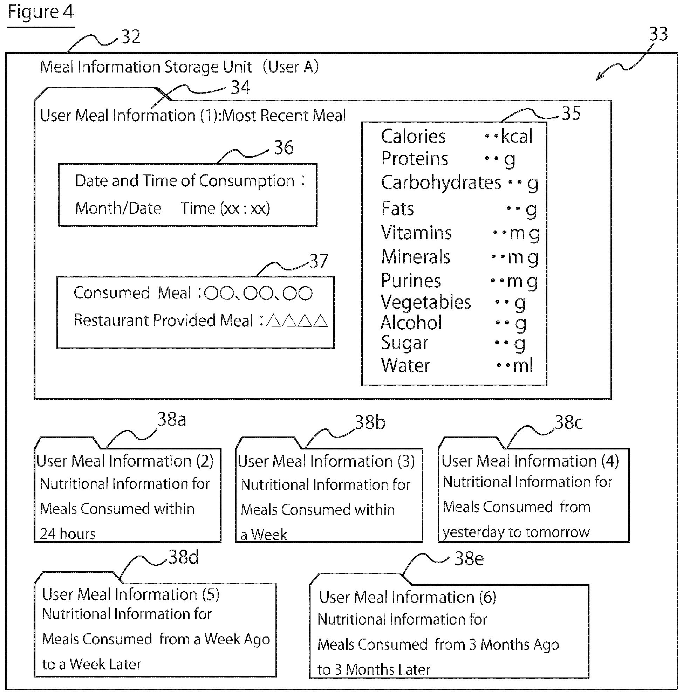

As shown in Figure 3, the meal information storage unit 32 of the user information management unit 30 stores user meal information 33 that includes information about the nutrition of dishes that a predetermined user has consumed in the past or plans to consume in the future, associated with the date and time or period of consumption 36. Figure 4 is a conceptual diagram explaining the information stored by the meal information storage unit 32.

As shown in Figure 4, the user information management unit 30 can store information about meals and their nutritional content consumed by a specific user in a format similar to a so-called diet log, categorized by meals such as breakfast, lunch, and dinner. The example shown in Figure 4 illustrates a state where the meal information storage unit 32 has stored user meal information (1) 34, which pertains to the most recent meal consumed by a specific user, as user meal information 33.The information stored as user meal information (1) 34 includes the date and time of consumption 36, meal and restaurant information 37 regarding the dishes consumed and the restaurants that provided them, and nutritional information 35 about the dishes included in that meal. Also, the date and time of consumption 36 may be integrated into user meal information 33 or managed separately from it. Furthermore, the information stored as user meal information 33 is not limited to the nutritional information of meals provided by restaurants; it may also include nutritional information about meals prepared and consumed by the user at home or other locations.

As shown in Figure 4, the nutritional information 35 about dishes consumed includes, for example, calories, proteins, carbohydrates, fats, vitamins, minerals, purines, vegetables, alcohol, sugar, salt, water, etc., contained in the consumed dishes, but are not limited to these. Also, user meal information 33 may include not only nutritional information about dishes the user has consumed in the past but also information about dishes the user plans to consume in the future. For example, if a specific user is planning to have a dinner course meal at a specific restaurant the following day, the meal information storage unit 32 may store the nutritional information of that dinner course meal as other user meal information, associating it with the date and time of consumption 36 (the following evening).

The user meal information 33 stored in the meal information storage unit 32 is not limited to nutritional information for individual meals, such as user meal information (1) 34 shown in Figure 4. It may also include cumulative nutritional information for multiple meals consumed within a specified intake period. For example, this could be nutritional information for meals consumed within 24 hours prior to the user searching for meals, as represented by user meal information (2) 38a. Additionally, the user meal information stored in the meal information storage unit 32 may include nutritional information for meals consumed within a week prior to the search, as represented by user meal information (3) 38b, or nutritional information for meals consumed from yesterday to tomorrow, as represented by user meal information (4) 38c.

Additionally, other types of user meal information 33 stored in the meal information storage unit 32 may include relatively long-term nutritional data, such as user meal information (5) 38d or user meal information (6) 38e. These could pertain to nutritional information for meals consumed over several weeks or months before and after the time the user searches for meals. By maintaining such long-term user meal information 33, the meal information storage unit 32 allows the information provision system 10 to retain highly useful nutritional data from the perspective of user health maintenance. Furthermore, with the support of the nutritional balance determination unit 40 and other features described later, users can plan their future meals and create schedules for their nutritional intake. This makes the information provision system 10 extremely beneficial for user health management. Additionally, if users develop the habit of planning future meals, it benefits the food service providers by making it easier to predict ingredient consumption, leading to advantages such as a reduction in food waste.

User meal information 33, such as user meal information (1) 34, can be input by users consuming meals via user terminals 92a, etc., but the meal information updating unit 78 of the server device 12 shown in Figure 2 can also update the user meal information 33. For example, the meal information updating unit 78 shown in Figure 2 can automatically update the user meal information 33 when a specific user utilizes a food service establishment managed by the store information management unit 20. This update is based on the food service establishment utilization information 29da (refer to Figure 5) obtained by the utilization information acquisition unit 76 and the dish nutritional information 23 stored in the dish information storage unit 22. The meal information updating unit 78 can identify the dishes consumed by the user and the date of consumption from the food service establishment utilization information 29da shown in Figure 5. By recognizing the nutritional details of those dishes from the dish nutritional information 23, it is able to generate and update the user meal information 33.

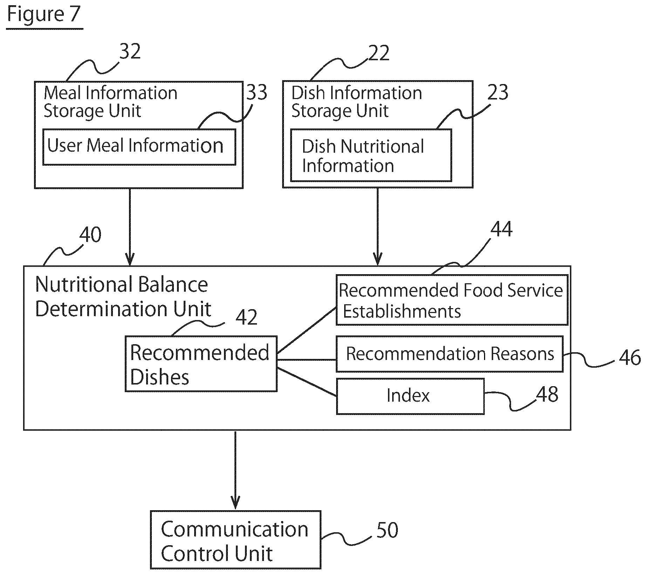

The nutritional balance determination unit 40 shown in Figure 2 uses the user meal information 33 (refer to Figure 4) and the dish nutritional information 23 (refer to Figure 6) to extract recommended dishes from those stored in the dish information storage unit 22. These recommendations are made from a nutritional perspective, identifying dishes that are suitable for the user associated with the user meal information 33. Figure 7 is a conceptual diagram illustrating the process by which the nutritional balance determination unit 40 extracts the recommended dishes 42.

As shown in Figure 7, the nutritional balance determination unit 40 begins processing when a signal requesting the search for recommended dishes is input from the user terminal 92a to the server device 12, as shown in Figure 1. The nutritional balance determination unit 40 reads the user's meal information 33 from the meal information storage unit 32 and understands the user's nutritional intake status. Based on this, the nutritional balance determination unit 40 determines, from a nutritional perspective, which nutrients should be supplemented or which nutrients should be reduced in intake. Then, the nutritional balance determination unit 40 identifies dishes from the dish nutritional information 23 stored in the dish information storage unit 22 that can effectively supplement the nutrients the user is lacking or prevent excessive intake of certain nutrients, thereby identifying the recommended dishes 42.

Furthermore, the nutritional balance determination unit 40 outputs the information of the recommended dishes 42, along with the information of the recommended food service establishments 44, which is the restaurant providing the recommended dishes, to the communication control unit 50. Additionally, the nutritional balance determination unit 40 may also calculate and output information related to the recommendation reasons 46, which explains the rationale for recommending the dish from a nutritional perspective, as well as information about an index 48 that indicates how suitable the recommended dish is for the user from a nutritional standpoint, along with the recommended dishes 42 and the recommended food service establishments 44 information, to the communication control unit 50.

Specific methods for extracting recommended dishes 42 by the nutritional balance determination unit 40 using the user meal information 33 and the dish nutritional information 23 are not particularly limited, but, for example, it can be a method where recommended dishes are those dishes that bring the intake of certain nutrients (e.g., calorie intake, purine intake, salt intake) within a predetermined period closer to their suitable values from a nutritional standpoint. Another method is to recommend dishes that contain a large amount of certain nutrients (e.g., "vegetables," "proteins," "vitamins," etc.) if a certain nutrient is found to be deficient based on past meal information. Another method is to recommend dishes that contain little to no amount of certain nutrients (e.g., "alcohol," "fats," etc.) or contain nutrients that help digest or break down excessive nutrients if it is found that certain nutrients are being excessively consumed based on past meal information.

Additionally, for example, if the user meal information 33 includes information about alcohol consumed in the past or planned in the future, the nutritional balance determination unit 40 can extract recommended dishes 42 to prevent the user's alcohol intake from becoming excessive within a predetermined period. Similarly, if the user meal information 33 includes information about calorie intake consumed in the past or planned in the future, the nutritional balance determination unit 40 can extract recommended dishes 42 to prevent the user's calorie intake from becoming excessive or insufficient within a predetermined period.

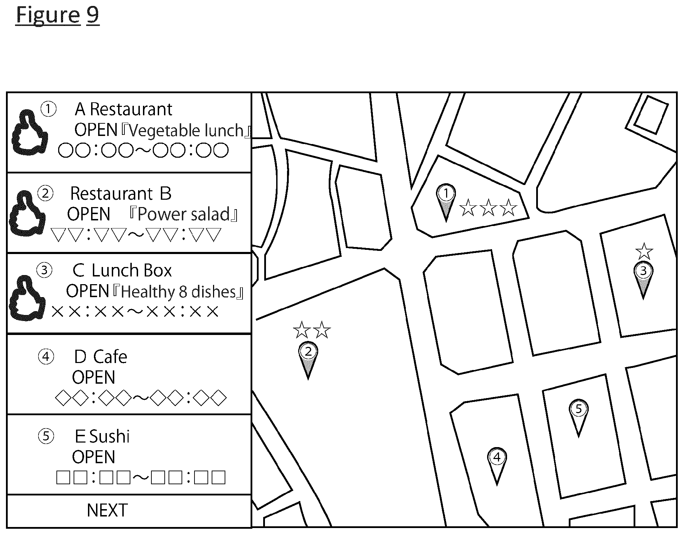

Furthermore, recommendation reasons 46 calculated by the nutritional balance determination unit 40 may include phrases like "low in calories," "rich in vegetables," "gentle on the body," etc., but are not limited to these. The index 48 calculated by the nutritional balance determination unit 40 may be a 3-stage or 5-stage rating expressed by the number of stars (see Figure 9) or a numerical rating expressed on a 10-stage or 100-stage scale, but are not limited to these.

Moreover, the nutritional balance determination unit 40 may use information other than the user meal information 33 and the dish nutritional information 23 when extracting recommended dishes 42. For example, it may obtain the user's location information 93 from the location information acquisition unit 70 shown in Figure 2, and read the geographical information 28a of food service establishments to limit recommended dishes 42 to those provided by establishments near the user's location.

Also, for example, the nutritional balance determination unit 40 may use, in addition to or instead of the user meal information 33 and the dish nutritional information 23, the user's body information 39a or meal preference related information 39c stored in the user information management unit 30 to extract recommended dishes 42. By considering body information 39a such as weight and basal metabolic rate, and meal preference related information 39c such as likes and dislikes, the nutritional balance determination unit 40 can extract recommended dishes 42 more suitable for specific users.

It should be noted that the nutritional balance determination unit 40, as shown in Figure 7, may extract a single recommended dish 42 and recommended food service establishments 44, or it may extract multiple recommended dishes 42 and recommended restaurants 44.

The communication control unit 50 shown in Figure 2 transmits information about the recommended dishes 42 and the recommended food service establishments 44 output by the nutritional balance determination unit 40 to the user's information terminal 92a. The communication control unit 50 may include signal conversion devices such as modems or ONUs, processors that control signal conversion devices and the input/output of signals, etc., but are not limited to these.

Also, as shown in Figure 7, when the nutritional balance determination unit 40 calculates recommendation reasons 46 or index 48, the communication control unit 50 may transmit information about the recommended dishes 42 and the recommended food service establishments 44 along with the recommendation reasons 46 and index 48 to the user's information terminal 92a. This allows users to know the recommendation reasons 46 and index 48 of the recommended dishes 42 and can enhance the motivation for users to consume the recommended dishes 42 from a nutritional standpoint.

Additionally, the communication control unit 50 can receive information about the recommended dishes 42 from the nutritional balance determination unit 40 and transmit it to the user terminal 92a. It can also read information related to the recommended food service establishments 44, such as operating hours, and details such as the price of the recommended dishes 42 from the dish information storage unit 22, and transmit this information to the user terminal 92a.

Moreover, the communication control unit 50 may transmit information about food service establishments other than the recommended food service establishments 44 within a predetermined range from the user's information terminal 92a. For example, if there are food service establishments other than recommended ones within a predetermined range from the user's location, the server device 12 can transmit their geographical information 28a to the user terminal 92a via the communication control unit 50.

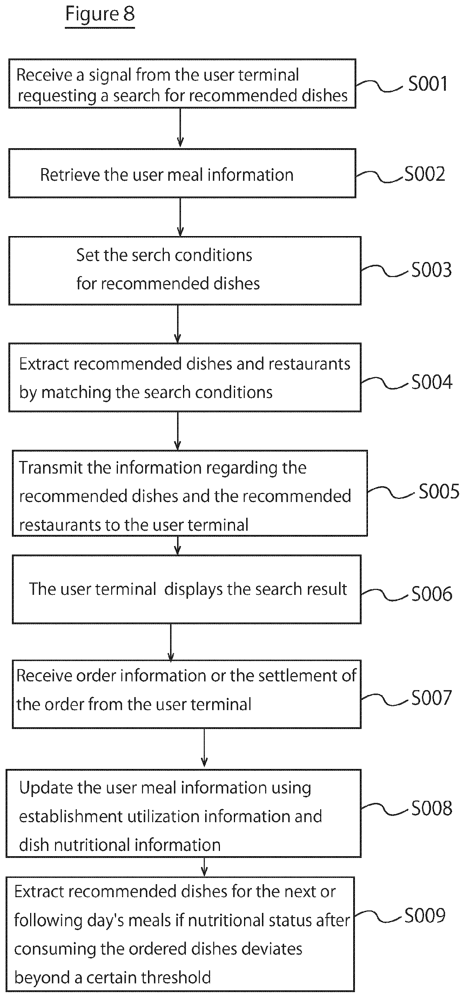

Figure 8 is a flowchart illustrating an example of the processing performed by the information provision system 10 for restaurants shown in Figure 1. In step 001 of Figure 8, the server device 12 receives a signal from the user terminal 92a requesting a search for recommended dishes. Upon receiving the search request for recommended dishes in step 001, the server device 12 initiates processing by having the nutritional balance determination unit 40, shown in Figure 7, retrieve the requesting user meal information 33 (refer to Figures 2-4) from the meal information storage unit 32 of the user information management unit 30.

In step S003, the nutritional balance determination unit 40 grasps the user's nutritional status based on the user meal information 33 and sets the conditions for recommended dishes 42 to be consumed in the user's next meal based on the user's nutritional intake status from a nutritional standpoint. At this time, the nutritional balance determination unit 40 may narrow down the search conditions for recommended dishes based on user settings, geographical information 28a of food service establishments, operating style information 29b, location information 93 of the user terminal 92a, and meal preference related information 39c.

In step S004, the nutritional balance determination unit 40 extracts recommended dishes 42 by matching the search conditions set in step S003 with the dish nutritional information 23 of dishes stored in the dish information storage unit 22 (see Figure 7). During the extraction of recommended dishes 42, the nutritional balance determination unit 40 can consider not only the dish nutritional information 23 but also geographical information 28a related to the restaurants providing the dishes and price information 26 of the dishes. This enables a more tailored search for dishes that best suit the user.

In step S005, the communication control unit 50, as shown in Figure 7, transmits the information regarding the recommended dishes 42 extracted in step S004 and the recommended food service establishments 44 offering those dishes to the user terminal 92a. This information is communicated along with recommendation reasons 46 and indices 48, as determined by the nutritional balance determination unit 40.

In step S006, the user terminal 92a (see Figure 1), which has received information about the recommended dishes 42 and related details from the server device 12, displays the search results. Figure 9 is a conceptual diagram illustrating an example of the search result display on the user terminal 92a. As shown in Figure 9, the display section of the user terminal 92a lists the recommended dishes 42, the recommended restaurants 44, and other food service establishments (on the left side of the screen). Additionally, the recommended food service establishments 44 and other food service establishments are displayed on a map of the relevant area.

Here, in step S005, when the server device 12 sends information to the user terminal 92a, the communication control unit 50 distinguishes the geographical information 28a of recommended food service establishments within a predetermined range from the geographical information 28a of non-recommended establishments and transmits them separately to the user terminal 92a. As a result, in step S006, as shown in the map of Figure 9, the geographical information (address) (1)-(3) of the recommended food service establishments is distinguished from the geographical information (address) (4)-(5) of non-recommended establishments. Furthermore, the communication control unit 50, in cases where at least one of the food service establishments within a specified range of the user terminal 92a's location is a recommended restaurant 44, distinguishes between the geographic information of the recommended food service establishments 44 and the geographic information of other non-recommended food service establishments. This information is then transmitted to the user terminal 92a.

In step S007, the server device 12 receives order information indicating the user's intention to order a dish or the settlement of the order from the user terminal 92a. For example, in Figure 9, the list of recommended food service establishments 44 and other establishments displayed can be expanded by selecting the corresponding section, and upon selecting recommended establishments from the list, the display section shows detailed information about the recommended dish 42 (such as price), details of other dishes, and information like business hours.

Furthermore, on a screen expanded from the list shown in Figure 9, icons or other elements for ordering the recommended dishes 24 offered by recommended food service establishments 44, etc., are displayed. By selecting these icons, the user can place an order for the recommended dish 24 with the recommended food service establishment 44, etc. In other words, the communication control unit 50 of the server device 12, shown in Figure 1, can receive order information 51 indicating the user's intention to order the recommended dish from the user terminal 92a. Furthermore, it can transmit the received order information 51 to the recommended food service establishment that provides the recommended dish (see Figure 1).

As a result, the food service establishment can receive the order information 51 from the server device 12 and prepare the ordered dishes quickly when the user arrives. Additionally, the user terminal 92a, as shown in Figure 1, allows not only placing food orders but also processing payments for the ordered dishes. Specifically, the communication control unit 50 of the server device can receive payment information 53, indicating the user's intent to complete the payment, along with the order information 51 (see Figure 1). Furthermore, the communication control unit 50 can transmit this payment information 53 to a designated payment institution 96 (refer to Figure 1). The payment institution 96 facilitates the payment process by collecting the fee from the user and transferring the payment to the restaurant, effectively mediating the user's payment to the food service establishment.

In Step S008, the utilization information acquisition unit 76 of the server device 12 retrieves food service establishment utilization information 29da (see Figure 5), which indicates that the ordered dish has been consumed by the user, based on the order information. Furthermore, in Step S008, the food service establishment utilization information 29da obtained by the utilization information acquisition unit 76 and the dish nutritional information 23 stored in the dish information storage unit 22 are used to update the user meal information 33 stored in the meal information storage unit 32. This allows the food service establishment information provision system shown in Figure 1 to automatically acquire and update the nutritional information of dishes the user has consumed in the past or plans to consume in the future, without requiring the user to manually input information about meals they have intentionally consumed.

In Step S009, based on the updated content of the user meal information 33 from step S008, the nutritional balance determination unit 40 evaluates the user's nutritional status after consuming the dishes ordered in Step S007. Furthermore, if the nutritional balance evaluation unit 40If it determines that the user's nutritional status after consuming the ordered dishes deviates beyond a certain threshold, it can extract recommended dishes for the next or following day's meals and transmit the information to the user terminal 92a via the communication control unit 50.

Such cases are likely to occur, for example, when the user orders a dish other than the recommended dish in Step S007. By ordering the recommended dish re-selected by the nutritional balance determination unit 40 in Step S009, the user can maintain an optimal nutritional balance over a longer period, even if they consumed a dish other than the recommended dish in their most recent meal.

Furthermore, with the food service establishment information provision system 10, users can be encouraged to place orders for meals to be consumed in future dining sessions, thereby contributing to the reduction of food waste at food service establishments. Additionally, the user meal information 33, which includes records of past meals and plans for future meals, can be accessed and retrieved from the server device 12 through the user terminal 92a. In this process, the user meal information 33 may be displayed on the display unit of the user terminal 92a in a calendar format based on the consumption dates 36. This allows users to easily check their meal records (including future reservations) through the food service establishment information provision system 10. Furthermore, some or all of the information managed by the user information management unit 30 shown in Figure 3, such as the user meal information 33, may be stored in the storage units of individual user terminals 92a, 92b, or 92c.

In the embodiment described above, the information provision system 10 for food service establishments has stored dish nutritional information 23 and stores information about dishes that users have consumed in the past or plan to consume in the future, and by using these, can provide users with information about diverse recommended dishes that are not uniform while taking into account the users' health. That is, the information provision system 10 can suggest healthier and more diverse meals by considering not only the nutritional information of a single meal but also the cumulative nutrition of multiple meals or over a longer period, thus securing the user's choice while promoting health. Additionally, by providing information about food service establishments and dishes that users have not visited or tried, the information provision system 10 can support users' diverse dietary lifestyles.

The above description has explained the food service establishment information provision system 10 with reference to the embodiment; however, the present invention is not limited to these embodiment alone and naturally includes numerous other embodiment and modifications. For instance, in the example shown in Figure 9, recommended food service establishments and non-recommended food service establishments are displayed distinctly. However, when displaying multiple recommended food service establishments, it is also possible to distinguish and display, on the map, those recommended food service establishments offering meals with higher indices among the multiple recommended establishments.

That is, when multiple recommended dishes 42 are extracted within a specified range from the location of the user terminal 92a, the communication control unit 50, as shown in Figure 7, transmits the geographic information of the recommended dining establishment offering the highest index 48 among the multiple recommended dishes 42 to the user terminal 92a, distinguishing it from the geographic information of other recommended dining establishments. This enables the user to easily identify the location of the recommended dining establishment providing the most optimal recommended dish from a nutritional perspective via a map or similar interface.

For example, the nutritional balance determination unit 40, as shown in Figure 7, can select the user meal information to be used for searching for recommended dishes 42 after obtaining the scheduled consumption date and time, which is the date and time the user plans to consume the recommended dishes 42. Specifically, when assessing the user's nutritional status using user meal information (1) 34, user meal information (2) 34a to user meal information (6) 34e, etc., stored in the meal information storage unit 32, the nutritional balance determination unit 40 can utilize nutritional information related to meals consumed within a specified period preceding the scheduled consumption date and time. This allows the nutritional balance determination unit 40 to predict the user's nutritional status at the time of consuming the recommended dish, even if the scheduled consumption date and time is in the relatively distant future (e.g., several days, weeks, or months). Based on this prediction, the unit can extract suitable recommended dishes 42.

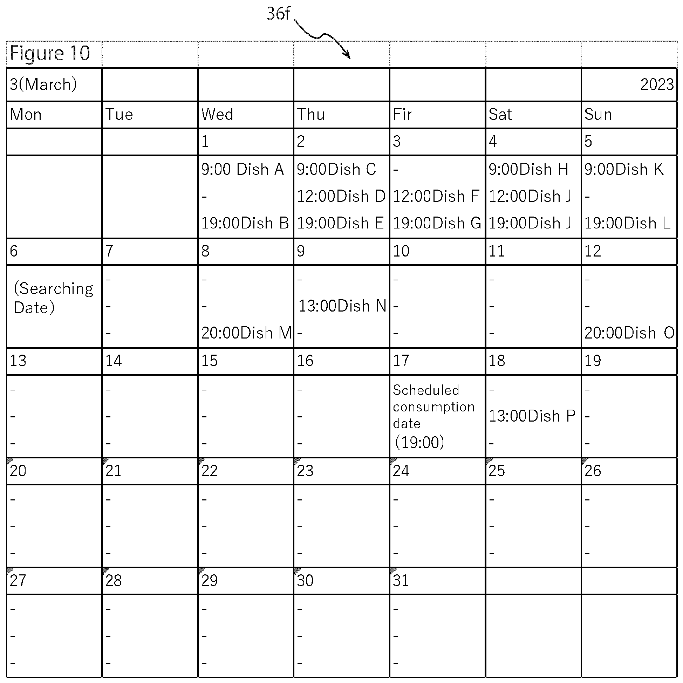

Also, for example, the nutritional balance determination unit 40 can extract recommended dishes by assessing the user's nutritional status based on user meal information 34 stored in the meal information storage unit 32. In doing so, it can set a reference date and time that corresponds to the same day of the week and time as the scheduled consumption date and time but on a different date. The unit can then utilize nutritional information related to meals consumed within a specified period prior to the reference date and time to extract suitable recommended dishes. Figure 10 shows another example of user meal information stored in the meal information storage unit 32, where a predetermined user has stored meal nutritional information 36f that includes information about dishes consumed in the past (before March 6) or planned in the future (after March 6) in a calendar format.

For example, as shown in Figure 10, if the scheduled consumption date and time is 7:00 PM on March 17, the nutritional balance determination unit 40 can set the reference date and time to 7:00 PM on March 3, which is the same day of the week and time as the scheduled consumption date and time but a different day, and perform extraction of recommended dishes based on meals consumed within a predetermined period before the reference date and time. Thus, even if there is little or no information recorded for the user's meal information 36f around the scheduled consumption date and time (March 17, 7:00 PM), the nutritional balance determination unit 40 can infer the user's nutritional status at the scheduled consumption date and time based on periodic trends of nutritional status and extract suitable recommended dishes.

For example, the nutritional balance determination unit 40, when assessing the user's nutritional status based on user meal information 34 or other data stored in the meal information storage unit 32, may set a reference date that corresponds to the same season and time as the scheduled meal date but on a different day. Using nutritional information related to meals consumed within a specified period before and after this reference date, the unit can extract recommended meals. For instance, the nutritional balance determination unit 40 could set a date exactly one year prior to the scheduled meal date as the reference date or use a specified day from the previous year or two years ago that falls within the same season as the scheduled date. This approach allows the nutritional balance determination unit 40 to predict the user's nutritional status for the scheduled meal date based on periodic trends in nutritional status, even if the user's meal information 40 around the scheduled date is missing or limited. As a result, the unit can extract meals that are optimal for the user.

Also, for example, the user information management unit 30 may store, as user body information 39a, age, weight, height, gender, basal metabolic rate, recommended daily calorie intake, BMI, blood pressure, body temperature, pulse, blood oxygen saturation, urine tests, blood tests, etc., including clinical test results such as sample tests, imaging tests, ECG tests, EEG tests, etc. The test values and grades included in the user body information 39a may include, for example, AST, ALT, γ-GTP, ALP, ChE, LDH, bilirubin, HBs antigen, HBs antibody, HCV antibody, total cholesterol, triglycerides, HDL cholesterol, LDL cholesterol, non-HDL cholesterol, blood glucose, HbA1c, uric acid, C-reactive protein, blood urea nitrogen, creatinine, eGFR, amylase, white blood cell count, red blood cell count, hemoglobin level, hematocrit, MCV, MCH, MCHC, platelet count, AFP quantification, CEA, prostate PSA, CA125, CA15-3, esterase, CYFRA, CA19-9, among others. The user information management unit 30 may obtain these test values and grades from the user terminal 92a or from wearable devices such as smartwatches or activity trackers that can communicate with the user terminal 92a.

The nutritional balance determination unit 40 may extract recommended dishes 42 using user meal information 33 and dish nutritional information 23, or alternatively, by utilizing user physical information 39a, such as BMI, blood pressure, body temperature, pulse rate, blood oxygen concentration, as well as test results and grades from urine or blood tests, and trends in these values. Furthermore, the nutritional balance determination unit 40 can calculate reference values for the cumulative nutrients that can be consumed over a specified period based on user physical information 39a, including BMI, blood pressure, body temperature, pulse rate, blood oxygen concentration, and test results or grades. It can then compare these reference values with information on the cumulative nutritional values of meals consumed over multiple occasions to extract recommended dishes 42. By doing so, the nutritional balance determination unit 40 can extract recommended dishes 42 that take into account the individual health conditions of each user. Moreover, by using user physical information 39a in addition to or instead of user meal information 33, the unit can extract suitable recommended dishes 42 even when user meal information 33 is insufficient or missing.

Also, for example, in the information provision system 10 for food service establishments shown in Figure 1, based on the user's preset settings, the communication control unit 50 can automatically place orders and perform settlements, and at a predetermined timing after ordering, automatically transmit information about the automatically ordered recommended dishes 42 and recommended food service establishments 44 to the user terminal 92a. For example, the information provision system 10 can automatically order and settle dishes for weekdays (on specified dates, multiple dates, or all weekdays) of the next month at a predetermined timing until the next month. In this case, the processing of the information provision system 10 is fundamentally the same as the sequence of processing explained in Figure 8, but step S001 is automatically started at a predetermined timing, steps S005 to S007 where the extraction results are communicated to the user terminal 92a are omitted, and the extracted recommended dishes 42 are automatically ordered by the communication control unit 50. Additionally, at a predetermined timing before the meal time (e.g., the day before the meal or one hour before the meal), the communication control unit 50 can transmit information about the automatically ordered recommended dishes 42 and recommended food service establishments 44 to the user terminal 92a to notify the user.

An information provision system 10 equipped with such an automatic ordering function can not only automatically manage the user's health but also promote reservation orders to restaurants, thereby potentially reducing food waste. It should be noted that the transmission of order information (including order information 51 and automatic order information) to recommended restaurants performed by the communication control unit 50, as shown in Figure 1, is not limited to transmission to servers managed by the recommended restaurants. It also includes transmission to order management servers that handle orders and related operations for the recommended restaurants.

### List of Reference Numerals

10···Information Provision System for Food Service Establishments
12···Server Device
14···Network Communication Network
20···Store Information Management Unit
22···Dish Information Storage Unit
22···Dish Nutritional Information
22···Dish
25···Nutrition
26···Price Information
27···Attribute Information
28···Regional Information Storage Unit
28a···Geographical Information
29a···Operating Hours Information
29b···Operating Style Information
29c···Real-time Information
29d···Customer Information
29da···Utilization Information
30···User Information Management Unit
32···Meal Information Storage Unit
33···User Meal Information
34···User Meal Information (1)
35···Nutritional Information about Consumed Dishes
36···Date and Time of Consumption
37···Consumed Dishes, Establishments
38a···User Meal Information (2)
38b···User Meal Information (3)
38c···User Meal Information (4)
39a···Body Information
39b···Regional Information
39c···Meal Preference Related Information
40···Nutritional Balance Determination Unit
42···Recommended Dishes
44···Recommended Food Service Establishments
46···Recommendation Reasons
48···Index
50···Communication Control Unit
51···Order Information
53···Payment Information
76···Utilization Information Acquisition Unit
78···Meal Information Updating Unit
79···Automatic Registration Support Unit
70···Location Information Acquisition Unit
92a···User Terminal
93···Location Information
94a···Food Service Establishment Terminal
96···Payment Institution

## Claims

1. An information provision system for food service establishments comprising:
A dish information storage unit that stores dishes provided by food service establishments associated with dish nutritional information, which is information about the nutrition of the dishes;
A meal information storage unit that stores user meal information, which includes information about the nutrition of dishes that a predetermined user has consumed in the past or plans to consume in the future, associated with the date and time or period of consumption;
A nutritional balance determination unit that uses the user meal information and the dish nutritional information to extract recommended dishes suitable for the user from the dishes stored in the dish information storage unit from a nutritional standpoint;
A communication control unit that transmits information about the recommended dishes and the recommended food service establishments providing these dishes to the user's information terminal;
wherein the user meal information includes information about the cumulative nutritional intake from multiple meals consumed by the user, and the nutritional balance determination unit grasps the user's nutritional intake status from the user meal information and finds dishes that can supplement the nutrients the user is lacking and prevent excessive intake of certain nutrients from the dishes stored in the dish information storage unit, thereby extracting the recommended dishes.

2. The information provision system for food service establishments according to claim 1, further comprising:
a utilization information acquisition unit that acquires utilization information, which indicates that the dishes stored in the dish information storage unit have been consumed by the user in the past or are scheduled to be consumed by the user in the future; and
a meal information updating unit that updates the user meal information stored in the meal information storage unit based on the utilization information and the dish nutrition information from the dish information storage unit.

3. The information provision system for food service establishments according to claim 1 or claim 2, further comprising:
a regional information storage unit that stores geographical information, including the addresses of the food service establishments and information about their surrounding geography; and
a location information acquisition unit that acquires the location information of the user's information terminal;
wherein the communication control unit, when at least one of the food service establishments within a specified range from the information terminal is identified as the recommended food service establishments, communicates the geographical information of the recommended food service establishments within the specified range to the user's information terminal, distinguishing it from the geographical information of food service establishments within the specified range that are not identified as recommended food service establishments.

4. The information provision system for food service establishments according to Claim 1, wherein the communication control unit receives order information indicating the user's intention to order the recommended dishes from the user's information terminal and transmits the order information to the recommended food service establishments providing the recommended dishes.

5. The information provision system for food service establishments according to Claim 4, wherein the communication control unit receives payment information indicating the user's intention to settle the order along with the order information and transmits the payment information to a predetermined payment institution.

6. The information provision system for food service establishments according to Claim 1, wherein the communication control unit transmits information about the recommended dishes and the recommended food service establishments along with information about the reasons for recommending the recommended dishes from a nutritional standpoint to the user's information terminal.

7. The information provision system for food service establishments according to Claim 1, wherein the communication control unit transmits information about the recommended dishes and the recommended food service establishments along with information about an index indicating the degree to which the recommended dishes are suitable for the user from a nutritional standpoint to the user's information terminal.

8. The information provision system for food service establishments according to Claim 7, further comprising:
A regional information storage unit that stores geographical information about the address and surrounding geography of food service establishments;
And a location information acquisition unit that acquires location information of the user's information terminal,
wherein the nutritional balance determination unit can extract multiple recommended dishes and recommended food service establishments,
and the communication control unit, if multiple recommended food service establishments are extracted within a predetermined range from the user's information terminal, transmits the geographical information of the recommended food service establishments with the highest index among them, distinguishing it from the geographical information of other recommended food service establishments.

9. The information provision system for food service establishments according to Claim 1, wherein the user meal information includes information about alcohol that the user has consumed in the past or plans to consume in the future.

10. The information provision system for food service establishments according to Claim 1, wherein the user meal information includes information about the calories of dishes that the user has consumed in the past or plans to consume in the future.

11. An information provision system for food service establishments comprising:
A dish information storage unit that stores dishes provided by food service establishments associated with dish nutritional information, which is information about the nutrition of the dishes;
A meal information storage unit that stores user meal information, which includes information about the nutrition of dishes that a predetermined user has consumed in the past or plans to consume in the future, associated with the date and time or period of consumption;
A nutritional balance determination unit that uses the user meal information and the dish nutritional information to extract recommended dishes suitable for the user from the dishes stored in the dish information storage unit from a nutritional standpoint;
And a communication control unit that transmits information about the recommended dishes and the recommended food service establishments providing these dishes to the user's information terminal,
wherein the user meal information includes information about the cumulative nutritional intake from multiple meals consumed by the user, and the nutritional balance determination unit obtains the scheduled consumption date and time of the recommended dishes,
and the nutritional balance determination unit sets a reference date and time different from the scheduled consumption date and time and uses the nutritional information of dishes consumed within a predetermined period to the reference date and time to extract the recommended dishes.

12. An information provision system for food service establishments comprising:
A dish information storage unit that stores dishes provided by food service establishments associated with dish nutritional information, which is information about the nutrition of the dishes;
A meal information storage unit that stores user meal information, which includes information about the nutrition of dishes that a predetermined user has consumed in the past or plans to consume in the future, associated with the date and time or period of consumption;
A nutritional balance determination unit that uses at least one of the user meal information and the user's body information and the dish nutritional information to extract recommended dishes suitable for the user from the dishes stored in the dish information storage unit from a nutritional standpoint;
And a communication control unit that transmits information about the recommended dishes and the recommended food service establishments providing these dishes to the user's information terminal,
wherein the user meal information includes information about the cumulative nutritional intake from multiple meals consumed by the user, and the nutritional balance determination unit grasps the user's nutritional intake status from the user meal information and/or the user's body information,
and finds dishes that can supplement the nutrients the user is lacking and prevent excessive intake of certain nutrients from the dishes stored in the dish information storage unit, thereby extracting the recommended dishes.

13. The information provision system for food service establishments according to any of Claims 1, 11, or 12, wherein the communication control unit based on the user's preset settings, automatically orders the recommended dishes extracted by the nutritional balance determination unit and sends the automatically ordered order information to the recommended food service establishments providing the recommended dishes.
